# EUROPEAN PATENT APPLICATION

(11) **EP 4 559 396 A1**
(43) Date of publication of application: **28.05.2025**
(21) Application number: 23212014.7
(22) Date of filing: 24.11.2023
(51) Int. Cl.: A61B 6/00, A61B 6/58

(54) **AUTOMATIC SYSTEM GEOMETRY ADJUSTMENT**

(71) Applicant: Koninklijke Philips N.V., 5656 AG Eindhoven (NL)
(72) Inventor: MENSER, Bernd, Eindhoven (NL); SENEGAS, Julien Thomas, Eindhoven (NL); DESHPANDE, Hrishikesh Narayanrao, 5656AG Eindhoven (NL); BERGTHOLDT, Martin, Eindhoven (NL); BYSTROV, Daniel, Eindhoven (NL); KROENKE-HILLE, Sven, Eindhoven (NL)
(74) Representative: Philips Intellectual Property & Standards

(57) **Abstract**

The present invention relates to radiation based imaging. In order to facilitate the adjustment of the system geometry of an X-ray system to a patient, there is provided a method (200) for controlling a movable device of an X-ray system to adapt the system geometry to a patient, the method comprising:
a) obtaining (210), at a first time, first image data of the patient from a sensor system;
b) determining (220) a first set of geometry parameters from the first image data, wherein the first set of geometry parameters is usable for controlling the movable device of the X-ray system to move to a first position;
c) obtaining (230), at a second time, second image data of the patient from the sensor system, wherein the first time is earlier than the second time, and a relative distance between the patient and the movable device of the X-ray system at the first time is larger than the relative distance between the patient and the movable device of the X-ray system at the second time; and
d) determining (240) a second set of geometry parameters from the second image data of the patient, wherein the second set of geometry parameters is usable for controlling the movable device of the X-ray system to move to a second position.

## Description

### FIELD OF THE INVENTION

The present invention relates to radiation based imaging and in particular to a method, to an apparatus, to a system, to a computer program element, and to a computer readable medium for controlling a movable device of an X-ray system to adapt the system geometry to a patient.

### BACKGROUND OF THE INVENTION

In the recent years, the moving parts of diagnostic X-ray systems, e.g., tube head or wall stand detector, have been motorized to facilitate the adjustment of the system geometry to the desired application and patient. Moreover, the system adjustment may be automatized using cameras and algorithms that analyze the scene and derive information for tailoring the system geometry parameters. However, there may exist conflicting requirements of patient-safety, real-time reaction, and best possible accuracy for adjusting the system geometry to a patient.

### SUMMARY OF THE INVENTION

There may be a need to facilitate the adjustment of the system geometry to a patient.

The object of the present invention is solved by the subject matter of the independent claims where further embodiments are incorporated in the dependent claims. It is understood that the following aspect of the invention equally applies to the method, to the apparatus, to the system, to the computer program element, and to the computer readable medium for controlling a movable device of an X-ray system to adapt a system geometry of the X-ray system to a patient.

According to a first aspect of the present invention, there is provided a method for controlling a movable device of an X-ray system to adapt a system geometry to a patient, the method comprising:
a) obtaining, at a first time, first image data of the patient from a sensor system;
b) determining a first set of geometry parameters from the first image data, wherein the first set of geometry parameters is usable for controlling the movable device of the X-ray system to move to a first position;
c) obtaining, at a second time, second image data of the patient from the sensor system, wherein the first time is earlier than the second time, and a relative distance between the patient and the movable device of the X-ray system at the first time is larger than the relative distance between the patient and the movable device of the X-ray system at the second time; and
d) determining a second set of geometry parameters from the second image data of the patient, wherein the second set of geometry parameters is usable for controlling the movable device of the X-ray system to move to a second position.

As discussed above, X-ray systems are being motorized and the geometry adjustment may be automatized, e.g. with the help of analysis of camera data. Such an automatized movement of the X-ray device must be done in a safe manner for the patient and instruments. The final recommended position should be as accurate as possible and the system should react instantaneously on the operator's request.

In order to address the conflicting requirements of patient-safety, real-time reaction, and best possible accuracy, the present disclosure proposes a two-stage approach with an initial coarse geometry adjustment based on the image data of the patient at some distance and a subsequent refinement, e.g., continuous refinement, with small steps based on the analysis of additional image data, e.g., successive camera frames. With the proposed two-stage approach, it is possible to avoid a large system movement in one go when the patient is close to the movable part of the X-ray system, thereby ensuring patient safety, better patient cooperation, and/or subsequently images with better diagnostic quality.

The term "system geometry" may also be referred to as imager geometry. A given system geometry may define the relative position between the patient, the X-ray detector, and the X-ray source. The system geometry may be represented by a set of angular or rectangular coordinate positions that describe a spatial configuration of the X-ray imaging system. The system geometry is dependent on the particular image equipment used but may comprise in general adjustable patient's table (if any), height, and position in space of the X-ray detector and X-ray source.

According to an exemplary embodiment of the first aspect of the present invention, the method further comprises the step of iteratively performing steps c) and d) while the relative distance between the patient and the movable device of the X-ray system decreases, until the movable device of the X-ray system is moved to a final position required for an X-ray image acquisition.

For the example of automatic detector height adjustment for wall stand chest exams, while the patient moves toward the detector to the final position required for the X-ray image acquisition, successive camera frames may be analyzed to obtain a final, stable and accurate estimate of the detector height.

According to an exemplary embodiment of the first aspect of the present invention, the step of iteratively performing step c) further comprises:
- applying temporal filtering to two or more successive image data of the patient acquired by the sensor system for determining a further set of geometry parameters, wherein the further set of geometry parameters is usable for controlling the movable device of the X-ray system to move to a further position.

Temporal filtering may mitigate the impact of noise in the input images or estimation results.

According to an exemplary embodiment of the first aspect of the present invention, the method further comprises the step of determining a change in patient position and/or a change in patient posture using the sensor system to determine a change in the relative distance between the patient and the movable device of the X-ray system.

For example, the sensor system may comprise one or more sensors configured to detect a proximity between the patient and the movable device of the X-ray system. Examples of the sensors may include, but are not limited to, an ultrasound sensor, an infrared sensor, a laser sensor, etc.

According to an exemplary embodiment of the first aspect of the present invention, the method further comprises the following steps:
- determining, based on a current patient position and/or the change in the relative distance between the patient and the movable device of the X-ray system, whether a planned geometry adjustment of the movable device of the X-ray system leads to a potential collision with the patient; and
- in response to determining that the planned geometry adjustment of the movable device of the X-ray system leads to a potential collision with the patient, modifying the planned geometry adjustment of the X-ray system to avoid the potential collision with the patient.

For example, depth-cameras or other suitable sensors may be used for estimating if the planned geometry adjustment would lead to a collision with the patient in his/her current position.

According to an exemplary embodiment of the first aspect of the present invention, the method further comprises the step of determining a patient motion based on the determined change in patient position and/or the determined change in patient posture. In step d) the second set of geometry parameters is determined from the second image data of the patient and the patient motion.

For example, the sensor signals can be used to algorithmically anticipate the change in the patient's position and/or posture in order to fine-tune the system geometry even faster.

According to a second aspect of the present invention, there is provided an apparatus for controlling a movable device of an X-ray system to adapt a system geometry to a patient, the apparatus comprising a processor configured to perform the steps of the method according to the first aspect and any associated example.

In some implementations, the apparatus may be provided as a personal computer, with a processor, for example an Intel (TM) 15 (TM), 17 (TM), or Xeon (TM) processor and the processor is configured to perform the functions via the loading of software instructions from non-volatile memory. The apparatus may have an input port capable of receiving image data from a sensor system. The input port may be provided, for example, as a USB interface, a Wi-Fi interface, an Ethernet interface, and the like. The apparatus may have an output port to output a control signal usable for controlling the movable device of the X-ray system. The output port may also be a USB interface, a Wi-Fi_33 interface, an Ethernet interface, and the like configured to communicate the control signal to the X-ray system. Of course, the output port may also comprise a graphics display card. For example, the planned system geometry adaption of the X-ray system may be displayed on a computer screen connected to the graphics display card.

In some other implementations, the apparatus may be embodied as, or in, an operator console. For example, the apparatus or some of its components may reside in an operator console running as software routines.

Specific embodiments of the apparatus will be explained subsequently.

Accordingly, and will be explained subsequently, an apparatus is provided that is capable of applying a two-stage approach to adapt a system geometry of an X-ray system to a patient. The two-stage approach includes an initial coarse geometry adjustment based on the image data of the patient at some distance and one or more subsequent refinements, e.g., continuous refinement, with small steps based on the analysis of additional image data, e.g., successive camera frames. Because it is possible to avoid a large system movement in one go when the patient is close to the movable part of the X-ray system, there is an opportunity to ensure patient safety, better patient cooperation, and/or subsequently images with better diagnostic quality.

According to a third aspect of the present invention, there is provided a system, which comprises a sensor system, an X-ray system configured to perform an X-ray image acquisition, and the apparatus according to the second aspect and any associated example configured to control a movable device of an X-ray system to adapt a system geometry to a patient using data obtained from the sensor system.

Accordingly, and as explained above with respect to the apparatus, the system is provided capable of controlling the X-ray system to adapt the system geometry to a patient with improved patient safety, better patient cooperation, and/or better diagnostic quality.

According to an exemplary embodiment of the third aspect of the present invention, the movable device of the X-ray system comprises an X-ray detector.

According to an exemplary embodiment of the third aspect of the present invention, the X-ray detector is a wall stand detector.

According to an exemplary embodiment of the third aspect of the present invention, the movable device of the X-ray system comprises a tube head.

According to an exemplary embodiment of the third aspect of the present invention, the sensor system comprises one or more of:
- a sensor for providing two-dimensional image data of the patient; and
- a sensor for providing surface data representing an outer surface of the patient.

In some examples, the sensor system may comprise a Red Green Blue (RGB) camera configured to provide two-dimensional image data of the patient.

In some examples, the sensor system may comprise a depth sensor configured to capture depth image data of the patient.

In some examples, the sensor system may comprise a sensor configured to capture image data stream indicative of one or more image data frames representing the patient.

With the captured two-dimensional image data and/or surface data, it is possible to detect the anatomic landmarks such as limbs, torso, neck, head, shoulder, overall body height, overall body width, etc. From the determined anatomic landmarks, the geometry parameters of the X-ray system may be determined, which are usable for controlling the movement of the movable device of the X-ray system.

In some implementations, the captured two-dimensional image data and/or the captured surface data may also be used to estimate a change of distance between the movable device and the patient.

According to an exemplary embodiment of the third aspect of the present invention, the sensor system further comprises a sensor for measuring a variation in distance between the patient and the movable device of the X-ray system.

In other words, in addition to the sensor for capturing the image data of the patient, the system may further comprise a sensor for measuring a distance between the patient and the movable device of the X-ray system. Using more than one sensor to measure a distance may provide benefits such as greater accuracy and a larger effective measurement range. For example, one or more embodiments may combine a sensor with a relatively low measurement range, but with fine resolution within this range for measuring the distance, with a sensor, e.g., a depth sensor, with a higher measurement range and a coarser resolution. This combination may for example provide highly accurate readings within the low range, but also provide valid data when the measured value exceeds the limited range of the fine resolution sensor.

Examples of the sensors may include, but are not limited to, an ultrasound sensor, an infrared sensor, a proximity sensor, etc.

According to another aspect of the present invention, there is provided a computer program product comprising instructions which, when the program is executed by a computer, cause the computer to carry out the steps of the method according to the first aspect and any associated example.

According to a further aspect of the present invention, there is provided a computer-readable storage medium having stored thereon the computer program product.

These and other aspects of the present invention will become apparent from and be elucidated with reference to the embodiments described hereinafter.

### BRIEF DESCRIPTION OF THE DRAWINGS

These and other aspects of the invention will be apparent from and elucidated further with reference to the embodiments described by way of examples in the following description and with reference to the accompanying drawings, in which
FIG. 1 is a schematic diagram illustrating a first perspective of an exemplary X-ray system.
FIG. 2 is a schematic diagram illustrating a second perspective of an exemplary X-ray system.
FIG. 3A is a schematic diagram illustrating that a patient stands with a first distance in front of an X-ray detector.
FIG. 3B is a schematic diagram illustrating that the patient stands with a second distance in front of the X-ray detector.
FIG. 4 illustrates a flow chart is shown for a method for controlling a movable device of an X-ray system to adapt a system geometry to a patient.

It should be noted that the figures are purely diagrammatic and not drawn to scale. In the figures, elements which correspond to elements already described may have the same reference numerals. Examples, embodiments or optional features, whether indicated as non-limiting or not, are not to be understood as limiting the invention as claimed.

### DETAILED DESCRIPTION OF EMBODIMENTS

In the following description, reference is made to an X-ray imaging system. In some example arrangements, the X-ray source of the X-ray imaging system is mounted to a ceiling via a gantry, and the corresponding X-ray detector is mounted to a stand and held in the vertical position. However, examples of the present disclosure are not limited to this particular arrangement, and it is to be appreciated that the X-ray source and X-ray detector may alternatively be mounted in a different manner, and also held in different positions. In addition, while reference is made to an X-ray detector, for instance, a wall stand detector as examples of movable device of the X-ray imaging system, it will be appreciated that the described method, apparatus, and system are also applicable to other movable devices, such as X-ray tube or tube head.

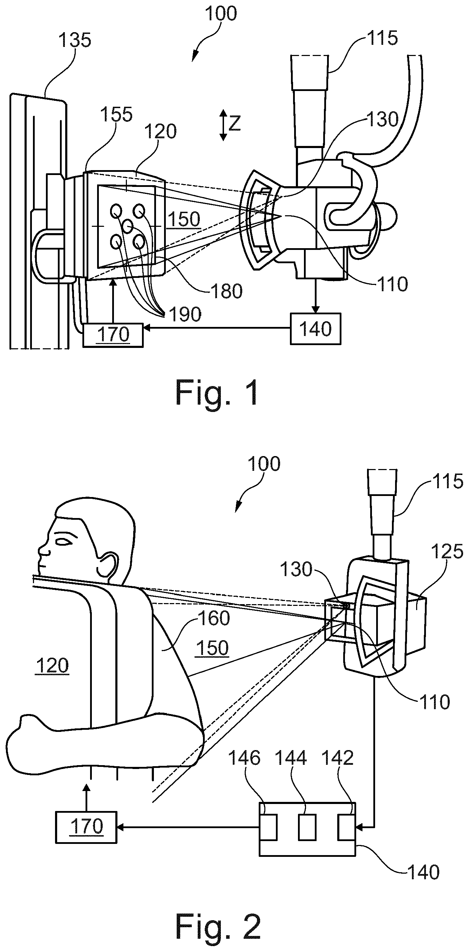

FIG. 1 is a schematic diagram illustrating a first perspective of an exemplary X-ray system 100. As shown in FIG. 1, the exemplary X-ray system 100 comprises an X-ray source 110 and an X-ray detector 120, which are separated by an examination region 150 for performing an X-ray imaging operation on a patient 160 (see FIG. 2) when the patient is located within the examination region 150. The X-ray source 110 and the X-ray detector 120 are movable so that an X-ray beam generated by the X-ray source 110 can be directed and adapted to a shape of a specific body part of the patient 160. For example, the patient's lungs may need to be examined in a "chest X-ray".

In the exemplary X-ray system 100 illustrated in FIG. 1, the X-ray source 110 is mounted to a celling via a telescopic arm 115, which may be affixed to an overhead carriage (not shown) that is slidable and movable along two sets of tracks (not shown). The telescopic arm 115 is arranged to allow movement of the X-ray source 110 up and down along a Z axis and rotation around same. The up/down movement of telescopic arm 115 and rotation around the Z axis may be effected by one or more motors (not shown).

Turning now to the X-ray detector 120, the X-ray detector 120 in the illustrated exemplary X-ray system 100 is mounted to a wall-stand 135 and held in the vertical position. The X-ray detector 120 comprises an X-ray radiation-sensitive region 180 and one or more radiation dose-measurement regions 190. In some implementations, the wall-stand 135 may be affixed to a floor and a wall. In some other implementations, the wall-stand 135 may be sufficiently rigid to be arranged as a free standing structure in the room without being affixed to a wall. In some further implementations, the X-ray detector 110 may be affixed to a celling (instead of floor or wall) and depends therefrom. As shown in FIG. 1, the X-ray detector 110 is mounted to a carriage 155, which is slidable in the Z direction in tracks integrated in the wall-stand 135. The Z direction is indicated with an arrow shown in FIG. 1. There may be arranged a motor 170, e.g., a stepper motor, that effects said movement along the z-axis of the X-ray detector 120. In some implementations, the carriage 155 may also be slidable on a separate track along an x-axis, such that the X-ray detector 120 can achieve a two-dimensional movement.

FIG. 2 is a schematic diagram illustrating a second perspective of an exemplary X-ray system 100. In FIG. 2, the patient 160 is close to his / her final position for an X-ray image acquisition. As shown in FIG. 2, during an image acquisition an X-ray beam emanates from X-ray source 110, passes through the patient 160 at a region of interest, experiences attenuation by interaction with matter therein, and the attenuated beam then strikes the X-ray detector 120. The X-ray detector 120 then issues a corresponding electric signal, which is translated by a data acquisition system (not shown) into a respective digital value representative of said attenuation. The density of the organic material making up the region of interest, e.g., rib cage and lung tissue in case of a lung x-ray, determines the level of attenuation. High density material, such as bone, causes higher attenuation than less dense materials, such as the lung tissue. The so registered digital values for the X-rays are then consolidated into an array of digital values forming an X-ray projection image for a given acquisition time and projection direction.

In the exemplary X-ray system shown in FIGs. 1 and 2, the movement of system parts, e.g., the X-ray detector 120, must be done in a safe manner, e.g. with some distance of the moving hardware to the patient. This is especially important for large motion, which may be required if the previous exam was done for a different anatomy. However, an accurate system positioning, e.g., detector height adjustment, may require the patient to be close to the final position, e.g., closer to the detector as shown in FIG. 2. Often, the geometry adjustment is done manually, i.e., fully controlled by the operator. Another approach for system positioning is to use a camera to automatically determine the geometry adjustment. In the exemplary X-ray system with a wall-stand X-ray detector shown in FIGs. 1 and 2, when the patient is close to his / her final position, a camera captures an image of the patient at his / her final position, and the system estimates the detector height accordingly. In other words, the adjustment of the detector height is done only once when the patient is close to his / her final position. The resulting detector height estimation could be prone to errors, e.g. due to noise in a single frame acquired by the camera used for the estimation. In both geometry adjustment approaches described above, the resulting displacement may be quite large e.g., a few centimeters. In that case, a few seconds may be needed for the detector to reach its target position, since the displacement speed of the detector needs to be slow for safety reasons. When an automatic geometry adjustment is triggered by the operator, e.g., by using a remote control, a prompt system reaction is expected without significant delay. This real time requirement may impose limits on the complexity of the scene analysis algorithm while still maintaining the desired accuracy. The detector height adjustment in this manner therefore induces source of inaccuracies, safety concerns, and unnecessary waiting time in the workflow.

In order to solve one or more of the above-described problems, an apparatus 140 is proposed for controlling a movable device of the X-ray system 100 to adapt a system geometry to the patient 160 based on data acquired by a sensor system 130. Therefore, a system comprising a sensor system 130, an X-ray system 100 configured to perform an X-ray image acquisition and the apparatus 140, as previously mentioned, configured to control a movable device of the X-ray system to adapt a system geometry to a patient using data obtained from the sensor system is also proposed. The sensor system 130 may comprise a sensor that is arranged on a housing 125 of the X-ray source 110. The sensor may be coupled to the apparatus 140 via a wired or wireless connection. In some examples, the sensor may be a camera that captures a two-dimensional image of the patient 160. In some examples, the sensor may be a range camera that is configured to acquire 3D image data of patient 160 by exposure of same to non-ionizing radiation or sound. The 3D image data is an array of pixels each pixel having a position in said array and a value. Each pixel position corresponds to a position of a point on the patient's surface and the value is directly related to or can be expressed in terms of a distance between camera's sensor and said patient surface point. Pixel values vary with sensor-object distance. Point cloud representation of the 3D image data set is also envisaged. In other words the 3D image data "follows" or describes the outer surface or perimeter of the patient 160 in 3D space. It is noted that the sensor is shown at the housing of the X-ray source 110 by way of example. It will be appreciated that the sensor may be arranged at any suitable position. In some examples, the sensor system 130 may comprise a sensor arranged on the ceiling, on the overhead carriage, on the wall, or on the wall-stand. In some implementations, the sensor may be positioned to capture essentially the entirety of the examination region 150 or at least that portion of the examination region 150 where the patient 160 can be expected to reside during the image acquisition.

In general, to effect the adaption of the system geometry, the sensor system 130 is configured to acquire image data of the patient 160 by exposure of the same to non-ionizing radiation or sound. The image data may include two-dimensional image data and/or three-dimensional image data. The acquired image data is then fed into the apparatus 140. The apparatus 140 then processes the image data of the patient in a manner described in more detail below to output a set of geometry parameters for controlling the movable device of the X-ray system 100 to adapt a system geometry to the patient 160. Broadly speaking, the apparatus 140 applies a two-stage approach to meet the conflicting requirements of patient-safety, real-time reaction, and best possible accuracy by doing a coarse geometry adjustment based on the image data of the patient at a first distance, and a subsequent refinement, e.g., continuous refinement, with small steps based on the analysis of successive frames. By avoiding the large system movement in one go when the patient is close to detector in this manner will ensure patient safety, better patient co-operation and subsequently images with better diagnostic quality.

Turning now to FIG. 2, this apparatus 140 may include an input port 142, a processing unit 144, and an output port 146. The apparatus 140 may be coupled to other devices, such as the sensor system 130 and a motor via a wired or wireless connection. The apparatus 140 may be implemented in numerous ways (e.g., such as with dedicated hardware) to perform the functions described herein. A "processor" is one example of the apparatus 140, which employs one or more microprocessors that may be programmed using software (e.g., microcode) to perform various functions described herein. The apparatus 140 may be implemented with or without employing a processor, and also may be implemented as a combination of dedicated hardware to perform some functions and a processor (e.g., one or more programmed microprocessors and associated circuitry) to perform other functions. Examples of apparatus components that may be employed in various embodiments of the present disclosure include, but are not limited to, conventional microprocessors, application specific integrated circuits (ASICs), and field-programmable gate arrays (FPGAs). In various implementations, a processor may be associated with one or more storage media (generically referred to herein as "memory," e.g., volatile and non-volatile computer memory). In some implementations, the storage media may be encoded with one or more programs that, when executed on one or more processors and/or controllers, perform at least some of the functions described herein. Various storage media may be fixed within the computing device or may be transportable, such that the one or more programs stored thereon can be loaded into the computing device so as to implement various aspects of the present disclosure described herein. The terms "program" or "computer program" are used herein in a generic sense to refer to any type of computer code (e.g., software or microcode) that can be employed to program one or more processors. As an example, the apparatus 140 or some of its components may reside in an operator console running as software routines. The components may be programmed in a suitable scientific computing platform such as Matlab^{®} or Simulink^{®} and then translated into C++ or C routines maintained in a library and linked when called on by the operator console.

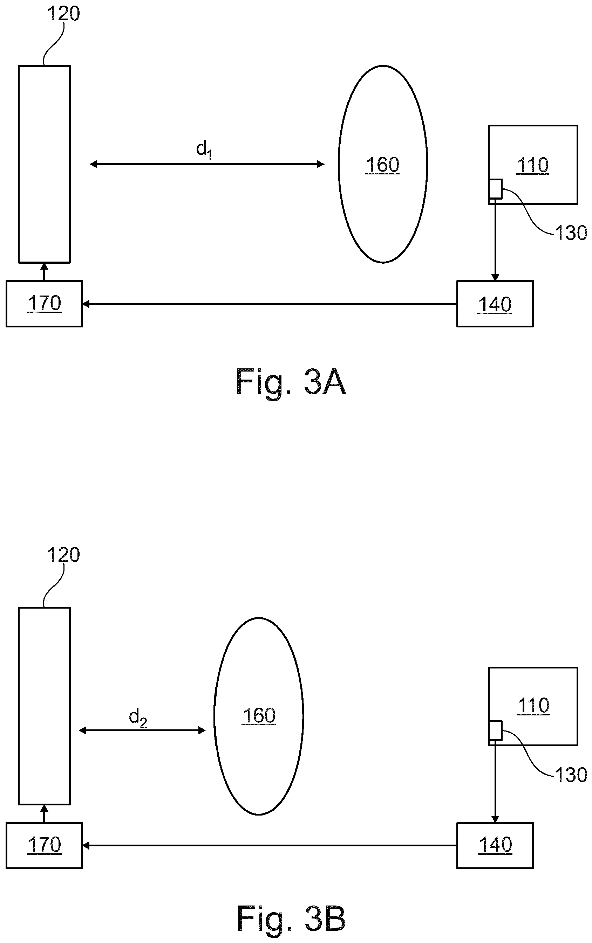

The operation of the apparatus 140 will be described with reference to FIGs. 3A and 3B, and described for the example of automatic detector height adjustment for wall stand chest exams as shown in FIGs. 1 and 2. In particular, FIGs. 3A and 3B illustrate that the patient 160 stands with different distances in front of the X-ray detector 120.

As shown in FIG. 3A, the patient 160 stands with a first distance d₁ in front of the X-ray detector 120. For example, an operator may ask the patient to stand with a safety distance, such as the distance d1 shown in FIG. 3A, in front of the X-ray detector 120. An automatic detector height adjustment may be triggered by the operator e.g., by using a remote control, or may be triggered by the sensor system 130 which detects a presence of the patient in front of the X-ray detector 120.

Either automatically or upon request by the operator, the apparatus 140 is configured to receive, via the input port 142, first image data of the patient 160 from the sensor system 130. The first image data may comprise 2D image data and/or 3D image data.

The first image data is then forwarded to the processing unit 144, which is configured to determine a first set of geometry parameters from the first image data. The set of geometry parameters may be a set of angular or rectangular coordinate positions that describe a spatial configuration of the X-ray imaging system. The imager geometry is dependent on the particular image equipment used but comprises in general adjustable patient's table (if any), height, and position in space of the X-ray detector and X-ray tube. Any given imager geometry defines the relative position between patient, X-ray detector, and the X-ray source. In the illustrated example, the set of geometry parameters comprises the height of the X-ray detector 120.

The processing unit 144 may determine the set of geometry parameters from image data in several ways. In some examples, one or more markers may be attached to the patient 160 e.g., to mark a region of interest. The sensor system 130 may capture an image of the patient to detect the position of the one or more markers and to determine the overall body height, and/ overall body width of the patient accordingly. In some other examples, no markers need be applied to the patient's body during the image acquisition. The patient walks into the examination room "as is" and towards a desired target spot therein. The sensor system 130 may capture 3D image data of the patient 160 showing 3D shape and geometry of the patient's 3D contours, which are then used to detect the anatomic landmarks such as limbs, torso, neck, head, shoulder, overall body height, overall body width, etc. Based on e.g., the height of the patient 160 and/or spatial position of a region of interest, the first set of geometry parameters is determined that includes a first height of the X-ray detector. A first control signal may be generated and outputted via the output port 146 for controlling the motor 170 to effect the movement along the z-axis of the X-ray detector 120 to a first position.

Since only a first coarse geometry adjustment is required, the algorithm can be optimized with respect to execution speed to enable a prompt reaction of the system to the operator trigger. The X-ray detector 120 is then automatically moved to the estimated position in response to the control signal. A higher displacement speed could be used at this stage.

As shown in FIG. 3B, the patient 160 moves toward a final position for the X-ray image acquisition. At a second time, the patient may move to a position with a second distance d₂ in front of the X-ray detector 120. The second distance d₂ is less than the first distance d₁. Either automatically or upon request by the imager operator, the apparatus 140 is configured to obtain, via the input port 142, second image data of the patient 160 from the sensor system 130. The second image data of the patient 160 is then forwarded to the processing unit 144, which is configured to determine a second set of geometry parameters from the second image data of the patient. In the illustrated example, the second set of geometry parameter may be determined that includes a second height of the X-ray detector. A second control signal may be generated and outputted via the output port 140 for controlling the motor 170 to effect the movement along the z-axis of the X-ray detector 120 to a second position.

In some implementations, the apparatus 140 may automatically and iteratively perform the above steps while the relative distance between the patient 160 and the movable device, e.g., the X-ray detector 120, of the X-ray system 100 decreases, until the movable device of the X-ray system is moved to a final position required for an X-ray image acquisition. For example, while the patient 160 moves toward the X-ray detector 120 to the final position required for the x-ray image acquisition, the sensor system 130 may capture successive camera frames, and the apparatus 140 may automatically and continuously analyze these camera frames to do small adaptions to the X-ray detector height. These continuous fine geometry adjustments may be executed rapidly without any harm to the patient 160. The continues fine geometry adjustments may be executed at a time interval of 5 seconds, 10 seconds, 15 seconds, or any suitable time interval. In some examples, the apparatus 140 may be configured to apply temporal filtering to two or more successive image data of the patient acquired by the sensor system for determining a further set of geometry parameters. The further set of geometry parameters is usable for controlling the movable device of the X-ray system to move to a further position. Temporal filtering may mitigate the impact of noise in the input images or estimation results.

According to an embodiment, the apparatus 140 may be configured to determine a change in patient position and/or a change in patient posture using the sensor system to determine a change in the relative distance between the patient and the movable device of the X-ray system. For example, the change in the relative distance may be estimated based on image data acquired by the sensor system. In some other examples, the sensor system 130 may additionally comprise a sensor for measuring a variation in distance between the patient and the movable device of the X-ray system. Such sensor may comprise a low range sensor, e.g., ultrasound sensors, infrared sensors, or other proximity sensors, for acquiring low range sensor data. In other words, in addition to the sensor for capturing the image data of the patient, the system may further comprise a sensor for measuring a distance between the patient and the movable device of the X-ray system. Using more than one sensor to measure a distance may provide benefits such as greater accuracy and a larger effective measurement range. For example, one or more embodiments may combine a sensor with a relatively low measurement range, but with fine resolution within this range for measuring the distance, with an image sensor with a higher measurement range and a coarser resolution. This combination may for example provide highly accurate readings within the low range, but also provide valid data when the measured value exceeds the limited range of the fine resolution sensor.

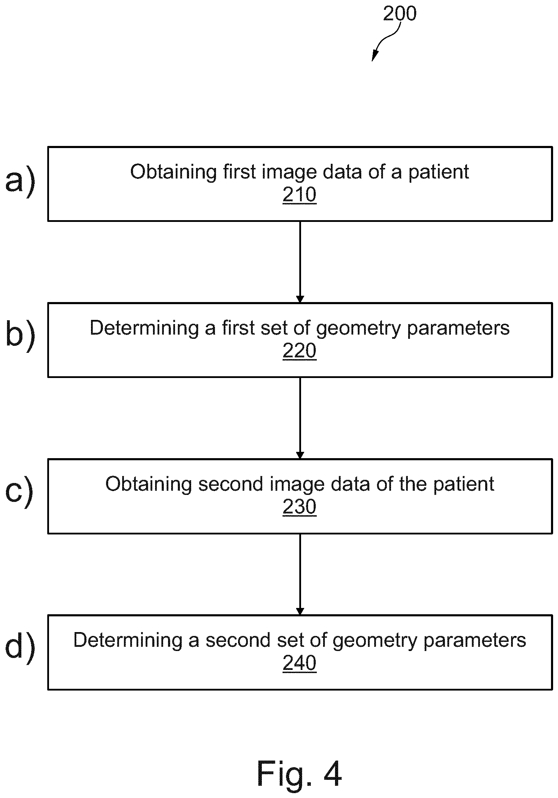

According to an embodiment, the apparatus 140 may be configured to determine, based on a current patient position and/or the change in the relative distance between the patient and the movable device of the X-ray system, whether a planned geometry adjustment of the movable device of the X-ray system leads to a potential collision with the patient. In response to determining that the planned geometry adjustment of the movable device of the X-ray system leads to a potential collision with the patient, the apparatus 140 may be configured to modify the planned geometry adjustment of the X-ray system to avoid the potential collision with the patient. For example, as explained above with respect to FIGs. 3A and 3B and as will be explained subsequently with respect to FIG. 4, the apparatus 140 may process the image data of the patient to determine a set of geometry parameters for controlling the movable device of the X-ray system 100 to move to a next position. Before sending a control signal to the motor 170 to effect such movement, the apparatus 140 may be configured to receive the current position of the patient and/or the change in the relative distance between the patient and the movable device of the X-ray system. The apparatus 140 may be further configured to determine whether the movable device, if moved to the next position (i.e., according to the planned geometry), would lead to a collision with the patient. For example, the apparatus 140 may be configured to determine whether the relative distance between the movable device at the next position and the patient is less than or equal to a threshold distance. In response to determining that the relative distance between the movable device at the next position and the patient is less than or equal to the threshold distance, the apparatus 140 may be configured to modify the set of geometry parameters for controlling the movable device of the X-ray system 100 to move to a different position, which would not lead to a collision with the patient. Afterwards, the apparatus 140 may be configured to generate a control signal to control the motor 170 to effect such movement of the X-ray detector 120.

According to an embodiment, the apparatus 140 may be configured to determine a patient motion based on the determined change in patient position and/or the determined change in patient posture. There are various sensors for detecting the patient motion, i.e., a change in the position of the patient 160 relative to its soundings. For example, infrared light or laser technology may be used for optical detection of patient motion. Motion may also be detected by monitoring changes in visible light using a video and camera system and in sound using an acoustic sensor. These sensor data is then fed into the apparatus 140, which is configured to process the sensor data to determine the patient motion. With the information about the patient motion, the apparatus 140 may anticipate the patient's position and/or posture at the next moment, and determine the second set of geometry parameters from the second image data of the patient and based on the patient's position and/or posture at the next moment in order to fine-tune the system geometry even faster. In other words, the sensor signals from the sensor system 130 may be used to algorithmically anticipate the change in the patient's position / posture in order to fine-tune the system geometry even faster.

In some implementations, the system geometry adjustment procedure may be automatic, in which the patient's presence detection is accomplished by the sensor system 130. In these implementations, the apparatus 140 may be in a stand-by mode and resume operation as soon as a patient 160 steps into the examination region 150. In this embodiment, a yellow line may be provided on the ground indicating a starting point, i.e., a first distance in front of the X-ray detector 120. When the patient stands with the first distance in front of the X-ray detector 120, the apparatus 140 receives first image data of the patient and determines a first set of geometry parameters for controlling the X-ray detector 120 to effect a first coarse geometry adjustment. As the patient moves towards the X-ray detector 120, the apparatus 140 receives one or more further image data of the patient, and generates one or more further control signals in order to fine-tune the system geometry. In a semi-automatic operation, the patient may be asked to step into the examination region 150. Once in the examination region 150, the operator may actuate an "enable" operation button or issue by other input means (touchscreen, mouse-click, pedal operation etc.) an "enable" signal to cause the apparatus 140 to start system geometry adjustment.

With reference to FIG. 4 a flow chart is shown for a method 200 for controlling a movable device of an X-ray system to adapt a system geometry to a patient.

At block 210, the method 200 comprises the step of a) obtaining, at a first time, first image data of the patient from a sensor system. The sensor system may comprise a sensor for providing 2D image data of the patient and/or a sensor for providing 3D image data representing an outer surface of the patient.

At block 220, the method 200 comprises the step of b) determining a first set of geometry parameters from the first image data. The first set of geometry parameters is usable for controlling the movable device of the X-ray system to move to a first position. For example, based on the first image data of the patient, e.g., from a camera mounted in the tube head, the height of the patient may be estimated, e.g. using an anatomical landmark detection algorithm. Since only a first coarse detector height estimation is required, the algorithm can be optimized with respect to execution speed to enable a prompt reaction of the system to the operator trigger. The detector is automatically moved to the estimated position. A higher displacement speed could be used at this stage.

At block 230, the method 200 further comprises the step of c) obtaining, at a second time, second image data of the patient from the sensor system. The first time is earlier than the second time, and a relative distance between the patient and the movable device of the X-ray system at the first time is larger than the relative distance between the patient and the movable device of the X-ray system at the second time.

At block 240, the method further comprises the step of d) determining a second set of geometry parameters from the second image data of the patient. The second set of geometry parameters is usable for controlling the movable device of the X-ray system to move to a second position. In other words, when the patient moves toward the detector to the final position required for the x-ray image acquisition, further image data is analyzed to obtain a further set of geometry parameters for controlling the movable device of the X-ray system to move to a second position.

In some implementations, the method 200 may further comprise the step of iteratively performing steps c) and d) while the relative distance between the patient and the movable device of the X-ray system decreases, until the movable device of the X-ray system is moved to a final position required for an X-ray image acquisition. For example, while the patient moves toward the detector to the final position required for the x-ray image acquisition, successive camera frames are being analyzed to obtain a final, stable and accurate estimate of the detector height. Temporal filtering can mitigate the impact of noise in the input images or estimation results. The system has to do only small adaptions to the detector height which can be executed rapidly without any harm to the patient.

In some implementations, the method 200 may further comprise the step of
determining a change in patient position and/or a change in patient posture using the sensor system to determine a change in the relative distance between the patient and the movable device of the X-ray system. In some examples, the change in patient position and/or a change in patient posture may be determined using the acquired 2D and/or 3D image data. In some examples, the change in patient position and/or a change in patient posture may be determined using other sensors, e.g., ultrasound or other proximity sensors that provide additional low range sensor data.

In some implementations, the method 200 may further comprise the step of determining, based on a current patient position and/or the change in the relative distance between the patient and the movable device of the X-ray system, whether a planned geometry adjustment of the movable device of the X-ray system leads to a potential collision with the patient. In response to determining that the planned geometry adjustment of the movable device of the X-ray system leads to a potential collision with the patient, modifying the planned geometry adjustment of the X-ray system to avoid the potential collision with the patient.

In some implementations, the method 200 may further comprise the step of determining a patient motion based on the determined change in patient position and/or the determined change in patient posture. In step d) the second set of geometry parameters is determined from the second image data of the patient and the patient motion.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100: X-ray system
- 110: X-ray source
- 115: telescopic arm
- 120: X-ray detector
- 125: housing of the X-ray source
- 130: sensor system
- 135: wall stand
- 140: apparatus
- 142: input port
- 144: processing unit
- 146: output port
- 150: examination region
- 155: carriage
- 160: patient
- 170: motor
- 180: X-ray radiation-sensitive region
- 190: one or more radiation dose-measurement regions
- 200: method
- 210: step a)
- 220: step b)
- 230: step c)
- 240: step d)

## Claims

1. A method (200) for controlling a movable device of an X-ray system to adapt a system geometry to a patient, the method comprising:
a) obtaining (210), at a first time, first image data of the patient from a sensor system;
b) determining (220) a first set of geometry parameters from the first image data, wherein the first set of geometry parameters is usable for controlling the movable device of the X-ray system to move to a first position;
c) obtaining (230), at a second time, second image data of the patient from the sensor system, wherein the first time is earlier than the second time, and a relative distance between the patient and the movable device of the X-ray system at the first time is larger than the relative distance between the patient and the movable device of the X-ray system at the second time; and
d) determining (240) a second set of geometry parameters from the second image data of the patient, wherein the second set of geometry parameters is usable for controlling the movable device of the X-ray system to move to a second position.

2. The method according to claim 1, further comprising:
- iteratively performing steps c) and d) while the relative distance between the patient and the movable device of the X-ray system decreases, until the movable device of the X-ray system is moved to a final position required for an X-ray image acquisition.

3. The method according to claim 2,
wherein the step of iteratively performing step c) further comprises:
- applying temporal filtering to two or more successive image data of the patient acquired by the sensor system for determining a further set of geometry parameters, wherein the further set of geometry parameters is usable for controlling the movable device of the X-ray system to move to a further position.

4. The method according to any one of the preceding claims, further comprising:
- determining a change in patient position and/or a change in patient posture using the sensor system to determine a change in the relative distance between the patient and the movable device of the X-ray system.

5. The method according to claim 4, further comprising:
- determining, based on a current patient position and/or the change in the relative distance between the patient and the movable device of the X-ray system, whether a planned geometry adjustment of the movable device of the X-ray system leads to a potential collision with the patient; and
- in response to determining that the planned geometry adjustment of the movable device of the X-ray system leads to a potential collision with the patient, modifying the planned geometry adjustment of the X-ray system to avoid the potential collision with the patient.

6. The method according to claim 4 or 5, further comprising:
- determining a patient motion based on the determined change in patient position and/or the determined change in patient posture;
wherein in step d) the second set of geometry parameters is determined from the second image data of the patient and the determined patient motion.

7. An apparatus (140) for controlling a movable device of an X-ray system to adapt a system geometry to a patient, the apparatus comprising a processor configured to perform the steps of the method of any one of the preceding claims.

8. A system, comprising:
- a sensor system (130);
- an X-ray system (100) configured to perform an X-ray image acquisition; and
- the apparatus (140) according to claim 7 configured to control a movable device of the X-ray system to adapt a system geometry to a patient using data obtained from the sensor system.

9. The system according to claim 8,
wherein the movable device of the X-ray system comprises an X-ray detector.

10. The system according to claim 9,
wherein the X-ray detector is a wall stand detector.

11. The system according to any one of claims 8 to 10,
wherein the movable device of the X-ray system comprises a tube head.

12. The system according to any one of claims 8 to 11,
wherein the sensor system comprises one or more of:
- a sensor for providing two-dimensional image data of the patient; and
- a sensor for providing surface data representing an outer surface of the patient.

13. The system according to claim 12,
wherein the sensor system further comprises a sensor for measuring a variation in distance between the patient and the movable device of the X-ray system.

14. A computer program element comprising instructions which, when the program is executed by a computer, cause the computer to carry out the steps of any one of claims 1 to 6.

15. A computer-readable storage medium having stored thereon the computer program product of claim 14.
